# EUROPEAN PATENT APPLICATION

(11) **EP 4 098 855 A1**
(43) Date of publication of application: **07.12.2022**
(21) Application number: 21202840.1
(22) Date of filing: 15.10.2021
(51) Int. Cl.: F01N 9/00, G01N 33/00

(54) **A DEVICE FOR MONITORING THE EMISSIONS OF A VEHICLE AND A KIT COMPRISING SUCH DEVICE**

(30) Priority: 01.06.2021 IT 202100014369
(71) Applicant: Claret S.r.l., 10146 Torino (TO) (IT)
(72) Inventor: Iannucci, Simone, 10146 Torino TO (IT); Solinas, Francesco Maria, 10146 Torino TO (IT)
(74) Representative: Perani & Partners S.p.A.

(57) **Abstract**

A vehicle emission monitoring device (1) comprises a main body (2) that can be fixed to a vehicle; detection means (4) connected to the main body (2) and adapted to collect emission data (D) representative of the instantaneous emissions of NOx and/or CO, and/or CO2, and/or PM10 from the vehicle; data communication means (6) located on the main body (2) and in signal communication with the detection means (4), the data communication means (6) is configured to send the emission data (D) to a receiving unit (10).

## Description

The present invention relates to a device for monitoring the emissions of a vehicle, and a kit comprising such a device. The device and the kit of the present invention can be used in the field of transportation, namely in both private and public road vehicles. By way of example, the monitoring device of the present invention may be used in cars, buses, vans, trucks and more. In particular, this monitoring device may be conveniently used to check the polluting emissions of internal combustion engines.

In recent times, increasing focus has been placed on environmental issues. In particular, many countries have been implementing programs for monitoring air quality in urban environments, which are known to be highly affected by the polluting emissions of road vehicles. Therefore, the values of the main pollutants in air is known to be monitored (by way of example, these pollutants can be NOx, CO, CO2 and PM10). Monitoring is carried out by means of fixed control units, located in predetermined locations and not easily displaceable.

The polluting emissions of vehicles are also known to be measured by means of special probes, located near the exhaust pipe. This measurement is made both during certification of vehicles by the relevant government agencies, i.e. before putting them on the market, and during subsequent monitoring, to check proper operation of the engine and ensure that the vehicle matches the emission standards in force.

While the causal link between vehicle emissions and air pollution is undoubtedly known, a close correlation between customary use of a single vehicle and local air pollution is hardly, if at all, determined. In other words, currently available systems cannot inform the vehicle driver about the contribution of the vehicle to air pollution, even though a strong correlation is known to exist between the driving style of a driver and the emissions of his/her vehicle.

### SUMMARY OF THE INVENTION

Therefore, the technical purpose of the present invention is to provide a device for monitoring the emissions of a vehicle, that can obviate the aforementioned prior art drawbacks.

In particular, the present invention has the object to provide a device for monitoring the emissions of a vehicle that can acquiring the emission data of a specific vehicle during its customary use.

The aforementioned technical purpose and objects are substantially fulfilled by a device for monitoring the emissions of a vehicle that comprises the technical features as disclosed in one or more of the accompanying claims.

In particular, this monitoring device comprises a main body designed to be fixed to a vehicle.

Detection means are connected to the main body, and are adapted to collect emission data representative of the instantaneous emissions of pollutants by the vehicle and/or environmental data representative of the concentration of pollutants in the air.

Data communication means are placed on the main body and in signal communication with the detection means. The data communication means is configured to send the emission data to a receiving unit.

The present invention further relates to a kit comprising the monitoring device. This kit also comprises a receiving unit adapted to be associated in signal communication with the data communication means of the monitoring device.

This device solves the technical problem as it can be mounted to a vehicle. Then, the data can be transmitted to the driver, who can account the actual emissions of pollutants and adapt his/her driving style.

Advantageously, the data can be also transferred to a remote collection center and, with an adequate number of devices, surveys can be conducted by correlating the emissions of each vehicle to the general level of air pollution in a given territory.

### LIST OF FIGURES

Further features and advantages of the present invention will result more clearly from the illustrative, non-limiting description of a preferred, non-exclusive embodiment of a monitoring device for vehicle emissions, as shown in the annexed drawings, in which:

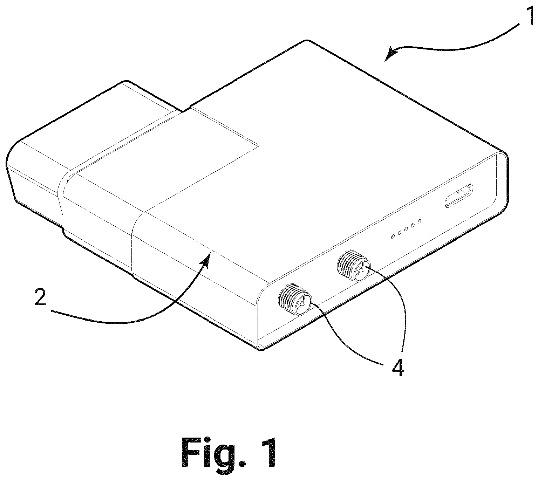

- Figure 1 is a perspective view of a device for monitoring the emissions of a vehicle of the present invention; and

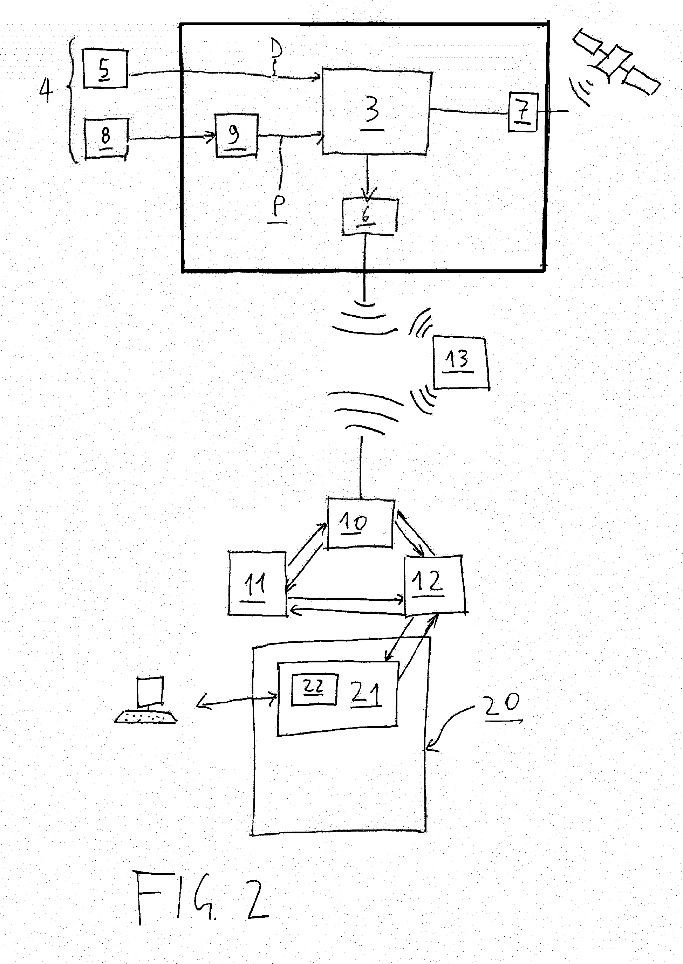

- Figure 2 is a block diagram that schematically shows the operation of the device of Figure 1, and the devices associated therewith.

### DETAILED DESCRIPTION

Referring to the annexed figures, numeral 1 designates a device for monitoring the emissions of a vehicle of the present invention.

In detail, the device 1 comprises a main body 2, which can be fixed to the vehicle (not shown). By way of example, the main body 2 may be arranged under the dashboard of the vehicle, connected to the "OBD" diagnostic port.

A processing unit 3 is housed in the main body 2. Further details about the data processing will be provided hereinbelow.

Detection means 4 are connected to the main body, and are in particular adapted to collect emission data "D" representative of the instantaneous emissions of pollutants such as NOx and/or CO, and/or CO2, and/or PM10 by the vehicle. In the preferred embodiment, the detection means 4 comprise a data port 8 that can be connected to the diagnostic port of the vehicle. The detection means 4 further comprise a data mining module 9, which is configured to calculate the emission data "D", namely by means of an artificial intelligence algorithm, from vehicle operating parameters. These parameters can be, for example, rpm and radiator temperature. Optionally, a representative value of the emission data "D" may be shown to the driver, to inform him/her about the environmental impact of his/her driving style.

Preferably, the detection means 4 comprise one or more probes 5 which, in use, are located outside the passenger compartment. Thus, the probe 5 is able to detect environmental data "P" representative of the concentration of one or more of the aforementioned pollutants. The probes 5 that are used are known per se and will not be further described.

The device 1 is further equipped with data communication means 6, arranged on the main body 2. These data communication means 6 interface in signal communication with the aforementioned detection means 4. The data communication means 6 are designed to send the emission data "D" and/or the environmental data "P" to a receiving unit 10.

It should be noted that the device 1 is equipped with a location module 7, which is configured to detect the position of the vehicle at the same time as the detection of the emission data "D". Namely, the location module 7 is designed to generate location data "L". More in detail, the location module 7 may comprise a satellite receiver, capable of receiving, for example, a GPS and/or GLONASS and/or Galileo signal. This satellite receiver is known per se and will not be further described herein.

More in detail, the aforementioned processing unit 3 is configured to associate the position of the vehicle, represented in particular by the location data "L", with the corresponding emission data "D" and/or with the environmental data "P".

The present invention further relates to a kit comprising the monitoring device 1. This kit also comprises a receiving unit 10, which can be associated in signal communication with the data communication means 6 of the monitoring device 1. This signal communication can occur by cable or, more preferably, by a wireless network, for example a WiFi, Bluetooth, 4G and/or 5G and/or LPWAN network. If transmission occurs with the "Low-Power Wide-Area Network" (LPWAN) protocol, data transmission will be facilitated by one or more repeaters 13 arranged in the urban environment in which the aforementioned vehicle circulates.

It should be noted that the kit may optionally comprise a navigation unit 11, which is associated with the device 1. In particular, the navigation unit 11, if any, may be incorporated in the device 1. Preferably, but without limitation, the receiving unit 10 and the navigation unit 11 may be implemented in software form in any device, for example in a smartphone or in a dedicated satellite navigator. In particular, the receiving unit 10 and the navigation unit 11 may be implemented by means of apps.

Advantageously, the navigation unit 11 may retrieve data from an external data source and suggest a navigation path that accounts for the air pollution levels associated with each section of the path and the emission data "D" detected by the device 1. For example, if emissions exceed a predetermined threshold, the navigation unit 11 may suggest a navigation route that avoids the most polluted areas of the town. Alternatively, the navigation unit 11 may exclude from the route certain areas in which pollution has to be reduced, for example near town parks or hospitals. For this purpose, the kit comprises a memory unit 12 associated with the receiving unit 10 and/or the device 1. This memory unit 12 can also be used to store the data required for the navigation unit 11. Advantageously, the memory unit 12 is arranged to store the emission data "D" detected by the device 1 and/or the environmental data "P". Finally, it should be further noted that the navigation unit 11 can also provide suggestions to the user to improve his/her driving style and reduce polluting emissions.

The present invention further relates to a system for monitoring air quality. This system can be implemented through a multitude of devices 1 and/or kits like those described above. Each device 1 and/or kit is associated with a respective vehicle.

This system comprises a data collection center 20, which is configured to anonymously receive the emission data "D" and/or the environmental data "P" of each vehicle sent by the respective devices 1. Namely, according to the preferred embodiment, in the system the emission data "D" and/or the environmental data "P" are anonymized, encrypted and stored in a distributed register, for example of the "blockchain" type. This data encryption system ensures transparency and reliability of the method used to calculate the emission data "P" associated with each vehicle. The data collected from each device 1 is stored on remote storage and/or a cloud.

Display means 21 are associated with the data collection center 20, and are configured to display the emission data "D" and/or the environmental data "P" retrieved from the data collection center 20. Preferably, the display means 21 comprises a platform that can be publicly accessed via a remote connection, for example via the Internet and/or the web. In particular, the display means 21 may be used by the navigation units 11 of the kits to provide the above described functions of defining and displaying the route.

More in detail, the display means 21 may comprise a map 22 associated with the platform. This map 22 may be advantageously configured to display the emission data "D" of all the vehicles having the device 1 preferably in real time or alternatively by selecting a predetermined time period.

## Claims

1. A device (1) for monitoring the emissions of a vehicle, **characterized in that** it comprises:
- a main body (2) designed to be fixed to a vehicle;
- detection means (4) connected to the main body (2) and adapted to collect emission data (D) representative of the instantaneous emissions of pollutants by the vehicle and/or environmental data (P) representative of the concentration of pollutants in the air;
- data communication means (6) located on the main body (2) and in signal communication with the detection means (4), the data communication means (6) being configured to send the emission data (D) to a receiving unit (10).

2. A device (1) as claimed in the preceding claim, **characterized in that** it comprises a location module (7) configured to detect the position of the vehicle while the emission data (D) and/or of the environmental data (P) are being collected.

3. A device (1) as claimed in the previous claim, **characterized in that** it comprises a processing unit (6) configured to associate the position of the vehicle with the emission data (D) and/or with the environmental data (P) being collected.

4. A kit comprising the monitoring device (1) as claimed in any of the preceding claims; a receiving unit (10) adapted to be associated in signal communication with the data communication means (6) of the monitoring device (1).

5. A kit as claimed in the preceding claim, **characterized in that** it comprises a navigation unit (11) associated with the monitoring device (1) and configured to provide a navigation path in response to air pollution levels associated with each section of the path and to the emission data (D) detected by the device (1).

6. A kit as claimed in the preceding claim, **characterized in that** it comprises a memory unit (12) associated with the receiving unit (10) and/or with the device (1), for storing the collected emission data (D).

7. A system for monitoring air quality, comprising a multitude of devices (1) as claimed in any of claims 1 to 3 and/or kits as claimed in any of claims 4 to 6.

8. A system as claimed in the preceding claim, **characterized in that** it comprises a data collection center (20) configured to associate the position of the vehicle with the emission data (D) and/or to receive the environmental data (P) of each vehicle in an anonymous form, preferably blockchain-based.

9. A system as claimed in claim 7 or 8, **characterized in that** it comprises display means (21) associated with the data collection center (20) and configured to display the emission data (D) collected from the data collection center (20).

10. A system as claimed in the preceding claim, **characterized in that** the display means (21) comprise a platform that can be publicly accessed via a remote connection, for example via the Internet and/or the Web; a map (22) associated with the platform and configured to display the emission data (D), preferably in real time.
